# FASCICULE DE BREVET EUROPEEN

(11) **EP 2 943 247 B1**
(45) Date de publication et mention de la délivrance du brevet: **20.06.2018**
(21) Numéro de dépôt: 14700391.7
(22) Date de dépôt: 14.01.2014
(51) Int. Cl.: A61B 5/00, A61B 5/053, A61N 1/04, A61N 1/08, A61N 1/30, A61N 1/32

(54) **DISPOSITIF D'ÉLECTROSTIMULATION ET/OU D'IONTOPHORÈSE AVEC DES MOYENS POUR FAIRE VARIER LA TENSION EN FONCTION DE LA RÉSISTIVITÉ DE LA PEAU D'UN UTILISATEUR**
GERÄT ZUR ELEKTROSTIMULATION UND/ODER ZUR IONTOPHORESE MIT MITTELN ZUR VARIATION DER SPANNUNG ALS FUNKTION DER RESISTIVITÄT DER HAUT EINES BENUTZERS
ELECTROSTIMULATION AND/OR IONTOPHORESIS DEVICE WITH MEANS FOR VARYING THE VOLTAGE AS A FUNCTION OF THE RESISTIVITY OF THE SKIN OF A USER

(30) Priorité: 14.01.2013 FR 1300061
(43) Date de publication de la demande: 18.11.2015
(73) Titulaire: Feeligreen, 06130 Grasse (FR)
(72) Inventeur: BIANCHI, Christophe, F-06100 Nice (FR)
(74) Mandataire: Brizio Delaporte, Allison
(86) Numéro de dépôt international: PCT/EP2014/050531
(87) Numéro de publication internationale: WO 2014/108548

(56) Documents cités:
- GB-A- 2 239 803
- GB-A- 2 411 961
- US-A- 5 823 989
- US-A1- 2012 316 456

## Description

### DOMAINE TECHNIQUE

La présente invention concerne un dispositif autorégulé d'électrostimulation et/ou iontophorèse, utilisable dans les techniques consistant à soumettre des cellules dermiques à un champ électrique pour augmenter leur activité et/ou à utiliser un courant électrique pour faciliter la diffusion transdermique de substances actives. La stimulation cellulaire par un champ électrique est utilisée dans de nombreuses applications. Ainsi l'électrostimulation décrite dans le brevet EP 0797421 sert à diminuer la douleur. L'électrostimulation peut également être utilisée pour le traitement des rides où on soumet les fibroblastes à un champ électrique pour augmenter leur taux de production de protéine, les protéines produites par les fibroblastes étant essentiellement le collagène et l'élastine et dont l'effet attendu est une amélioration de l'élasticité de la peau et donc un effet visible de réduction des rides. L'électrostimulation peut aussi être utilisée pour le traitement de la cicatrisation pour laquelle le champ électrique augmente la migration cellulaire et facilite ainsi la cicatrisation.

L'iontophorèse telle que décrite dans le brevet EP 1727593 est une technique qui peut être utilisée à des fins cosmétiques et/ou médicales pour introduire des substances actives dans le derme de la peau au moyen d'un courant. Les substances actives qui se trouvent dans un réservoir ou dans un hydrogel sont généralement composées de molécules de complexité variable ayant des masses molaires importantes comprises entre 10.000 et 1.000.000 g.mol-1.

### ETAT DE LA TECHNIQUE

L'application d'un courant électrique par l'intermédiaire d'un timbre à la surface de la peau pendant une période de temps relativement longue, comme c'est le cas dans l'électrostimulation ou l'iontophorèse, peut provoquer une modification des caractéristiques de la peau, voire des lésions allant d'un oedème superficiel à des brûlures profondes.

Des solutions proposées dans l'état de l'art mesurent au début du traitement des paramètres physiologiques de la peau à l'endroit du timbre et des données liées à l'utilisateur pour adapter la tension délivrée par le dispositif. Toutefois, des lésions sont encore présentes. Le document GB 2 239 803 A décrit un timbre applicable sur la peau d'un utilisateur et des moyens pour faire varier la tension en fonction de la résistivité de la peau de l'utilisateur. Il existe le besoin de proposer un dispositif qui améliore la sécurité de tels dispositifs pour supprimer les risques de lésion tout en maintenant une efficacité de l'électrostimulation et/ou de l'iontophorèse.

### RESUME DE L'INVENTION

C'est pourquoi, le but principal de l'invention est de fournir un dispositif d'électrostimulation et/ou iontophorèse qui s'adapte en temps réel aux changements de la peau de façon à ne pas faire courir de risques et en particulier des risques de brûlures thermiques ou chimiques dues à des densités de courant trop importantes à son utilisateur.

L'objet principal de l'invention est donc un dispositif d'électrostimulation et/ou iontophorèse comprenant un timbre adapté pour être appliqué sur la peau d'un utilisateur et un générateur de tension adapté pour appliquer un courant de faible intensité à travers la peau de l'utilisateur par l'intermédiaire d'un système d'électrodes se trouvant dans le timbre. Ce dispositif comprend un autorégulateur qui fait varier la tension fournie par le générateur de tension en fonction de la résistivité de la peau de l'utilisateur sous le timbre, la résistivité calculée en temps réel. Le demandeur s'est aperçu que la calculation de la résistivité en temps réel en tant qu'une grandeur représentative de l'état de la peau indépendante des caractéristiques du dispositif de mesure, permettait un contrôle en temps réel du courant fourni et donc de limiter voire supprimer tout risque de brûlure.

Un deuxième objet de l'invention est un dispositif d'électrostimulation et/ou iontophorèse dans lequel le système d'électrodes comprend une pluralité d'électrodes d'une première polarité connectées à une des bornes du générateur de tension et une pluralité d'électrodes de la seconde polarité connectées à l'autre borne du générateur de tension, les électrodes d'une première polarité étant disposées de façon alternée avec les électrodes de la seconde polarité et les électrodes se trouvant aux deux extrémités du timbre étant de même polarité.

Un troisième objet de l'invention est un dispositif d'électrostimulation et/ou iontophorèse dans lequel chacune des électrodes, anode ou cathode, comprend une pluralité de points de contact de manière à répartir le courant appliqué sur plusieurs interfaces et donc de diviser la densité de courant locale.

### BREVE DESCRIPTION DES DESSINS

Les buts, objets et caractéristiques de l'invention apparaîtront plus clairement à la lecture de la description qui suit faite en référence aux dessins dans lesquels :
- la figure 1 représente de façon schématique un dispositif d'iontophorèse comportant un autorégulateur selon l'invention ;
- la figure 2 est un organigramme des étapes mises en oeuvre par l'autorégulateur durant l'application d'un traitement par le dispositif d'électrostimulation et/ou iontophorèse selon l'invention; et
- la figure 3 représente un timbre utilisé pour la mise en oeuvre d'un dispositif d'électrostimulation et/ou iontophorèse selon l'invention.

### DESCRIPTION DETAILLEE DE L'INVENTION

Avant d'entamer une revue détaillée des modes de réalisation de l'invention, sont énoncées ci-après des caractéristiques optionnelles qui pourront éventuellement être utilisées en association ou alternativement.

Avantageusement, la présente invention concerne un dispositif d'électrostimulation et/ou iontophorèse comprenant un timbre adapté pour être appliqué sur la peau d'un utilisateur et un générateur de tension adapté pour appliquer un courant de faible intensité à travers la peau de l'utilisateur par l'intermédiaire d'un système d'électrodes se trouvant dans ledit timbre ; ledit dispositif comprenant un autorégulateur qui fait varier la tension fournie par ledit générateur de tension en fonction de la résistivité de la peau de l'utilisateur sous ledit timbre, ledit autorégulateur comprenant un élément de mesure de la résistance de la peau la valeur de la résistance permettant audit autorégulateur de calculer en temps réel la valeur de la résistivité de la peau de l'utilisateur sous ledit timbre.

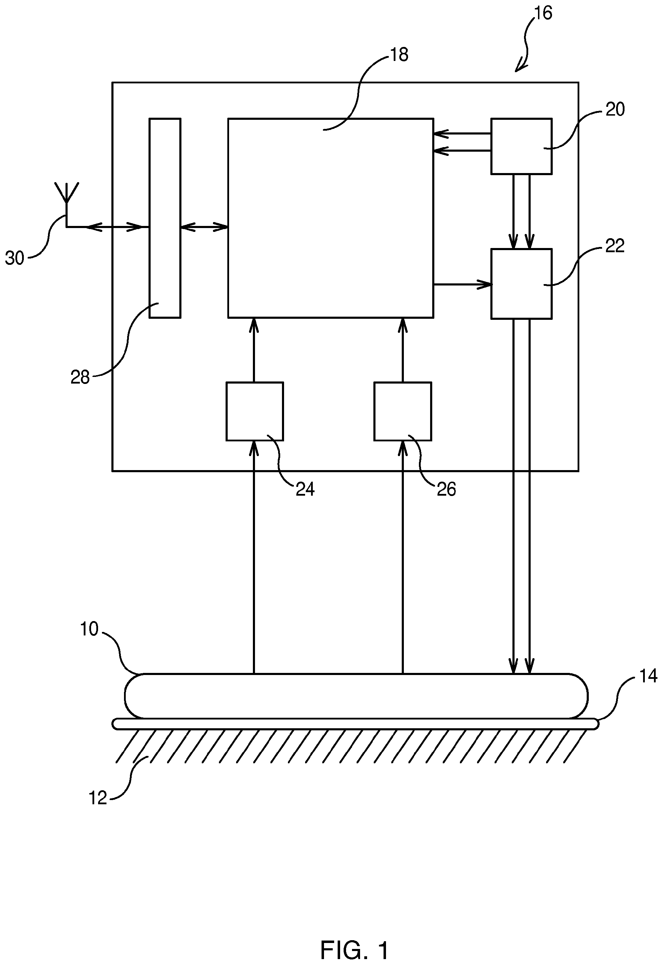

En référence à la figure 1, le dispositif selon l'invention comprend un timbre 10, appelé communément « patch », qui est appliqué sur la peau 12 de l'utilisateur au moyen d'un produit adhésif.

A noter que la figure 1 représente un dispositif d'iontophorèse et que, dans ce cas, le timbre 10 n'est pas directement appliqué sur la peau, mais par l'intermédiaire d'une couche 14 contenant des substances actives comme on le verra par la suite. Si le dispositif est utilisé simplement comme dispositif d'électrostimulation, le timbre 10 est appliqué directement sur la peau de l'utilisateur.

Selon la caractéristique essentielle, le dispositif comprend un autorégulateur 16. Ce dernier comprend un microprocesseur 18 alimenté par une source d'énergie électrique 20 telle qu'un accumulateur rechargeable ou une pile et qui commande l'application d'une tension à des électrodes se trouvant dans le timbre 10 par l'intermédiaire d'un générateur de tension 22.

Le microprocesseur 18 reçoit des informations provenant avantageusement de deux entrées. La première entrée est fournie par un élément de mesure de résistance 24 et avantageusement la seconde entrée est fournie par un élément de mesure de la température 26.

A noter que, comme il a déjà été mentionné, la résistance de la peau est une grandeur mesurable qui dépend des caractéristiques du dispositif de mesure. Par contre, la résistivité est une grandeur représentative de l'état de la peau indépendante des caractéristiques du dispositif de mesure. C'est pourquoi l'élément de mesure 24 détermine la résistance de la peau, ce qui est facile à réaliser, et le microprocesseur 18 calcule la résistivité de la peau, seule valeur significative de l'état de la peau, en utilisant les caractéristiques de l'élément de mesure qui lui ont été fournies préalablement.

En plus de la résistivité de la peau, il faut avantageusement tenir compte de la température de la peau fournie par l'élément de mesure de température 26 dans la mesure où la tension à appliquer doit également tenir compte de cette température. En outre, il est préférable pour un bon traitement que cette température ne dépasse pas une température prédéterminée.

A partir de la valeur de la résistivité obtenue, le microprocesseur 18 calcule la tension à appliquer de façon à ce que cette tension soit toujours en deçà d'un seuil pouvant provoquer des lésions ou brûlures.

Le demandeur a identifié la résistivité comme un paramètre sûr et fiable pour la surveillance de l'état de la peau au niveau du timbre notamment pour limiter les risques de lésions et/ou brûlures. De manière surprenante, la résistivité est une grandeur qui varie en fonction du courant fourni et de la peau. Les variations de la résistivité se sont avérées rapides et de fortes amplitudes. La mesure de la résistivité permet d'établir en retour la tension à appliquer qui suit alors immédiatement les variations de la résistivité et donc du courant fourni. Le dispositif permet une sécurité accrue pour l'utilisateur sans baisse de l'efficacité. Le courant fourni est toujours adapté à l'instant.

Le dispositif selon l'invention permet un contrôle en continu de la résistivité. Ce contrôle en continu est particulièrement avantageux car il permet de contrôler le courant fourni en continu et donc d'avoir une fiabilité et une sécurité du dispositif accrue. On entend par continu une fréquence de mesure inférieure à la seconde.

Le présent dispositif contrôle la densité du courant fourni mais également la profondeur de pénétration du courant.

Le dispositif selon l'invention est donc particulièrement fiable.

L'autorégulateur 16 comprend également une interface de communication/programmation 28 lui permettant de recevoir de l'extérieur la configuration à utiliser comprenant les paramètres à utiliser, et de communiquer à l'extérieur les résultats du traitement mis en place. Dans le mode de réalisation préférentiel, l'interface 28 est connectée au moyen d'une antenne 30 à un ordinateur, téléphone portable au autre dispositif équivalent grâce à une liaison « Bluetooth ».

A noter que le générateur de tension 22 peut fournir, sous la commande du microprocesseur 18, soit une tension continue, soit une tension alternative, soit une tension pulsée, dépendant du traitement à appliquer.

Une tension pulsée est particulièrement avantageuse car elle permet de limiter l'oxydation de l'électrode et ainsi la migration des composants de l'électrode à la peau. En effet, ce transfert peut être particulièrement néfaste au vue des durées d'utilisation longues de ce type de dispositif.

En outre, l'autorégulateur 16 est alors adapté en conséquence pour mesurer la résistance en temps réel de la peau, ce qui est particulièrement délicat dans ces conditions. Il est préféré de recourir à un micro-échantillonnage. En effet, la résistivité peut être différente à la fin d'une impulsion et au début de la suivante. La résistivité doit donc être mesurée à une fréquence élevée afin de pouvoir réagir rapidement, notamment dès le début de l'impulsion. La fréquence lors d'un micro-échantillonnage est supérieure à 1kHz.

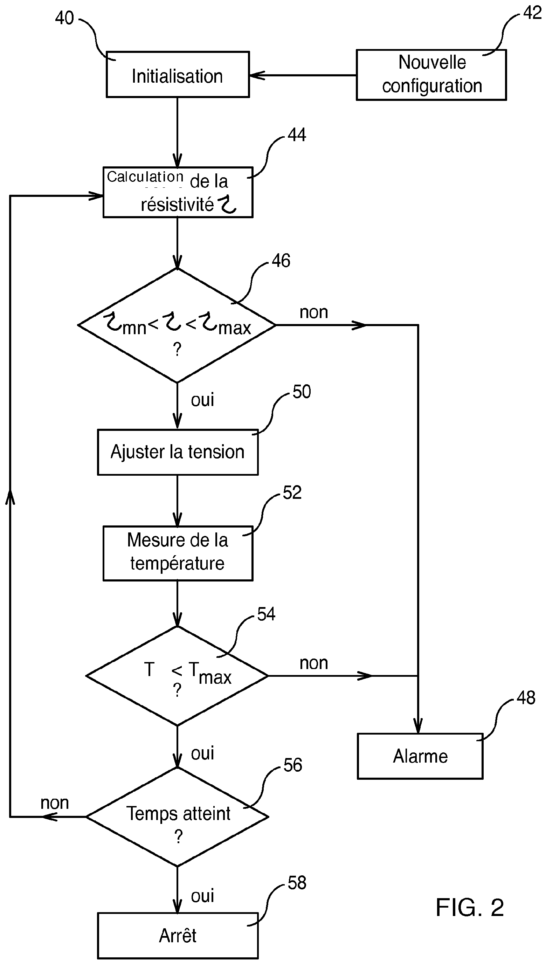

En référence à la figure 2 qui illustre l'organigramme des étapes suivies par le logiciel de l'autorégulateur, on commence par initialiser l'autorégulateur (étape 40) en lui fournissant une nouvelle configuration (étape 42) par l'interface 28 qui comporte les caractéristiques du traitement à effectuer telles que la tension à appliquer et la durée du traitement.

Puis l'autorégulateur effectue la calculation de la résistivité r (étape 44) au moyen de l'élément de mesure 24. On détermine alors si la résistivité de la peau est comprise entre une valeur minimale rₘᵢₙ et une valeur maximale rₘₐₓ (étape 46). En effet, une valeur très faible de la résistivité signifierait que le système est en court-circuit et une valeur trop élevée impliquerait une tension trop élevée faisant courir des risques de brûlures à l'utilisateur.

Si la valeur de la résistivité n'est pas comprise entre rₘᵢₙ et rₘₐₓ, une alarme est déclenchée (étape 48), alarme qui consiste en l'allumage d'un voyant lumineux (non montré). Si la valeur de la résistivité est comprise entre ces deux valeurs, la tension est ajustée à la valeur désirée (étape 50).

Puis l'autorégulateur effectue la mesure de la température T de la peau (étape 52) au moyen de l'élément de mesure 26, ce qui permet de déterminer si cette température est inférieure à une température prédéterminée Tₘₐₓ (étape 54). En effet, une température de la peau trop élevée est un indicateur de risques de brûlures chimiques et une limitation de la température permet également d'avoir une sécurité supplémentaire dans le cas d'un traitement de longue durée, par exemple pour traiter les escarres. Si la température de la peau dépasse la température Tₘₐₓ, une alarme est déclenchée (étape 48).

Lorsque la température de la peau n'est pas supérieure à Tₘₐₓ, le test suivant consiste à déterminer si le temps imparti au traitement a été atteint (étape 56). Si ce n'est pas le cas, le processus reboucle à la mesure de la résistivité (étape 44). Si c'est le cas, le processus s'arrête (étape 58).

Un paramètre important du dispositif d'électrostimulation et/ou iontophorèse qui fait l'objet de l'invention est que la densité de courant traversant l'épiderme doit être inférieure à une densité prédéterminée estimée à 11 mA/cm². En effet, si la densité de courant appliqué était supérieure à cette valeur, il y aurait des risques de brûlures.

C'est pourquoi, selon un deuxième objet de l'invention, le dispositif comprend une pluralité d'anodes et une pluralité de cathodes alternées avec les anodes pour que le courant total se divise suivant plusieurs circuits anode-cathode de manière à ce que la densité du courant circulant d'une anode à une cathode soit inférieure à 1 mA/ cm². En outre, afin d'empêcher la formation de lignes de champ externes (à l'extérieur du dispositif), les deux électrodes se trouvant aux deux extrémités sont de même polarité, c'est à dire que ce sont deux anodes ou deux cathodes.

Un dispositif multi-électrodes présente en outre un avantage important, à savoir que la distance entre une anode et une cathode doit être inférieure à une distance prédéterminée fonction de la tension appliquée. En effet, il est important que les lignes de courant ne pénètrent pas dans l'hypoderme de manière à éviter de provoquer des microlésions musculaires ou tendineuses par voie directe ou angio-ischémique. C'est pourquoi la distance entre l'anode et la cathode doit être inférieure à une distance prédéterminée fonction de la tension appliquée. Ainsi, pour une tension de 5V, la distance entre l'anode et la cathode doit être inférieure à 2cm.

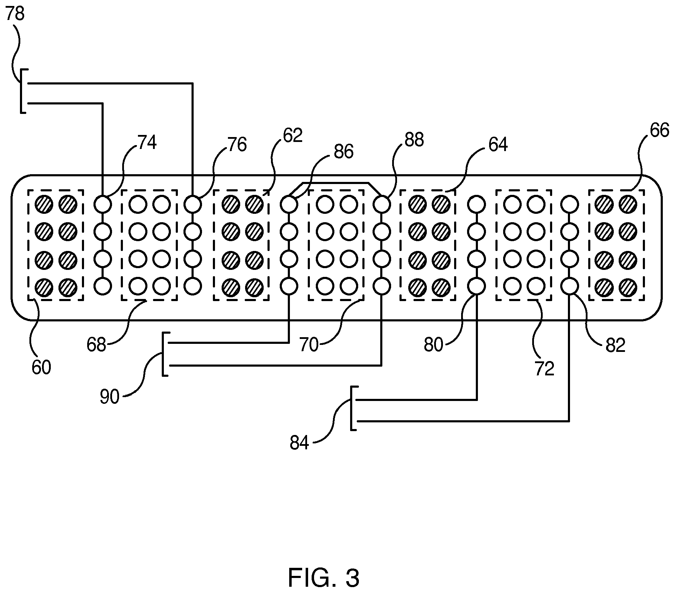

Ainsi, la figure 3 représente un timbre de grandes dimensions (utilisé par exemple pour le front ou le cou) ayant une longueur d'environ 14cm comportant 4 anodes 60, 62, 64 et 66 alternées avec 3 cathodes 68, 70 et 72, les 2 électrodes d'extrémité du timbre étant 2 anodes comme il a été mentionné ci-dessus.

Comme mentionné ci-dessus, la distance entre 2 électrodes est inférieure ou égale à une distance prédéterminée, par exemple 2cm pour une tension appliquée de 5V. Pour la mesure de la résistance, le dispositif illustré sur la figure 3 comporte des ensembles de points de contact 74 et 76 connectés par les connexions 78 à l'élément de mesure 24. Les points de contact 74 sont situés à mi-chemin entre l'anode 60 et la cathode 68, et les points de contact 76 sont situés à mi-chemin entre la cathode 68 et l'anode 62. A noter que cette mesure peut se faire par tout moyen approprié tel qu'un pont de Wheatstone.

Dans le mode de réalisation illustré sur la figure 3, un deuxième ensemble de points de contact est utilisé, à savoir les points de contact 80 situés à mi-chemin entre l'anode 64 et la cathode 72, et les points de contact 82 situés à mi-chemin entre la cathode 72 et l'anode 66 connectés à l'élément de mesure 26 par les connexions 84, ceci dans le but d'obtenir une valeur moyenne plus représentative de la résistance de la peau.

La mesure de la température de la peau se fait par les ensembles de points de contact 86 et 88 connectés par les connexions 90 à l'élément de mesure de la température 26. De façon préférentielle, les ensembles de points de contact sont des thermistances.

Dans le mode de réalisation préférentiel illustré sur la figure 3, chaque électrode, anode ou cathode, est une électrode multipoint comportant 8 points de contact de façon à mieux répartir le courant appliqué à travers la peau. Chaque point de contact est en un alliage or-nickel et a une surface de 5 mm². L'intérêt d'utiliser des électrodes multipoint découle du fait que l'épiderme de la peau n'est pas une surface lisse et qu'une simple électrode étant peu flexible, elle risque de se détacher au moins partiellement de la peau et ainsi provoquer la création de mini arcs électriques ou de brûlures chimiques de l'épiderme par une augmentation de la densité de courant au point de contact.

L'intensité du courant appliqué dans le cas de la figure 3 se répartit sur 3 cathodes, chacune étant constituée de 8 points de contact. Par conséquent, l'intensité I du courant appliqué doit se conformer à l'inégalité I/3 ≤ 8x0,05, soit I ≤ 1,2mA.

Comme mentionné précédemment, si le dispositif est utilisé pour l'électrostimulation, le timbre directement appliqué sur la peau sert à faire pénétrer le courant dans la peau pour, soit stimuler les fibroblastes de manière à augmenter leur taux de production de collagène et d'élastine dont l'effet est une amélioration de l'élasticité de la peau, soit traiter une cicatrisation.

Lorsque le dispositif est utilisé pour l'iontophorèse, il comporte une couche 14 sous le timbre 10 et contenant les substances actives à faire pénétrer dans le derme, telles que la vitamine A ou rétinol (dépigmentant), l'acide rétinoïque (traitement de l'acné), la vitamine C (antioxydant), un chélateur d'ions tel que l'acide β-alanine di-acétique (pour le traitement des érythèmes), l'acide glycolique (amélioration de la texture de la peau), le phosphate sodique de dexaméthasone (anti-inflammatoire) ou tout autre type d'actif ionisé ou présenté sous forme d'émulsions anioniques ou cationiques afin de pouvoir être véhiculé par le courant.

Selon un mode de réalisation préférentiel, la couche 14 est un hydrogel qui présente le double avantage de ne pas nécessiter de réservoir (pour un liquide) et d'être flexible de façon à épouser le contour de la peau sur laquelle il est appliqué.

Bien que le dispositif illustré sur la figure 3 comprenne le même nombre de points de contact pour chaque électrode, il va de soi que ce nombre pourrait être différent sans sortir du cadre de l'invention. De même, les points de contact de chaque électrode pourraient être disposés différemment que selon des rectangles.

## Revendications

1. Dispositif d'électrostimulation et/ou iontophorèse comprenant un timbre (10) adapté pour être appliqué sur la peau (12) d'un utilisateur et un générateur de tension (22) adapté pour appliquer un courant de faible intensité à travers la peau de l'utilisateur par l'intermédiaire d'un système d'électrodes se trouvant dans ledit timbre ; ledit dispositif comprenant un autorégulateur (16) qui fait varier la tension fournie par ledit générateur de tension en fonction de la résistivité de la peau de l'utilisateur sous ledit timbre, ledit dispositif étant **caractérisé en ce que** ledit autorégulateur (16) comprend un élément de mesure de la résistance de la peau (24) et **en ce que** ledit autorégulateur est adapté à utiliser la valeur de la résistance pour calculer en temps réel la valeur de la résistivité de la peau de l'utilisateur sous ledit timbre.

2. Dispositif selon la revendication précédente, ledit dispositif étant adapté à déclencher une alarme lorsque la valeur de la résistivité de la peau n'est pas comprise entre une valeur minimale et une valeur maximale

3. Dispositif selon l'une quelconque des revendications précédentes, dans lequel ledit autorégulateur (16) comprend un élément de mesure de la température de la peau (26). étant adapté à

4. Dispositif selon la revendication 3, ledit dispositif étant adapté à déclencher une alarme lorsque la valeur de la température de la peau dépasse une valeur maximale.

5. Dispositif selon l'une quelconque des revendications précédentes, dans lequel ledit système d'électrodes comprend une pluralité d'électrodes d'une première polarité (60, 62, 64, 66) connectées à une des bornes dudit générateur de tension et une pluralité d'électrodes de la seconde polarité (68, 70, 72) connectées à l'autre borne dudit générateur de tension, lesdites électrodes d'une première polarité étant disposées de façon alternée avec lesdites électrodes de la seconde polarité et les électrodes se trouvant aux deux extrémités dudit timbre étant de même polarité.

6. Dispositif selon la revendication 5, dans lequel la distance entre une anode et une cathode adjacente est inférieure à une distance prédéterminée de deux centimètres pour que les lignes de courant ne pénètrent pas dans l'hypoderme de manière à éviter de provoquer des microlésions musculaires ou tendineuses.

7. Dispositif selon l'une quelconque des deux revendications précédentes, dans lequel chacune desdites électrodes, anode ou cathode, comprend une pluralité de points de contact de manière à répartir le courant appliqué sur plusieurs interfaces et donc de diviser la densité de courant locale.

8. Dispositif selon l'une quelconque des revendications 1 à 7, utilisable comme dispositif d'iontophorèse et comportant, sur une surface dudit timbre (10) destinée à être appliquée sur la peau de l'utilisateur, une couche de substances actives (14) introduisibles avec le courant dans la peau de l'utilisateur.

9. Dispositif selon la revendication 8, dans lequel ladite couche de substances actives est sous forme d'hydrogel.

## Patentansprüche

1. Vorrichtung zur Elektrostimulation und/oder lontophorese, umfassend ein zum Aufbringen auf die Haut (12) eines Anwenders angepasstes Pflaster (10) und einen zum Aufbringen eines Stroms geringer Intensität durch die Haut des Anwenders mittels eines sich in dem Pflaster befindlichen Elektrodensystems angepassten Spannungsgenerator (22); wobei die Vorrichtung einen Selbstregler (16) umfasst, der die durch den Spannungsgenerator gelieferte Spannung als Funktion des Widerstands der Haut des Anwenders unter dem Pflaster variiert, wobei die Vorrichtung **dadurch gekennzeichnet ist, dass** der Selbstregler (16) ein Messelement des Widerstands der Haut (24) umfasst, dadurch, dass der Selbstregler zum Verwenden des Wertes des Widerstands zum Berechnen des Widerstandswerts der Haut des Anwenders unter dem Pflaster in Echtzeit angepasst ist.

2. Vorrichtung nach dem vorstehenden Anspruch, wobei die Vorrichtung zum Auslösen eines Alarms angepasst ist, wenn der Widerstandswert der Haut nicht im Bereich zwischen einem Minimalwert und einem Maximalwert liegt.

3. Vorrichtung nach einem der vorstehenden Ansprüche, wobei der Selbstregler (16) ein Messelement der Temperatur der Haut (26) umfasst.

4. Vorrichtung nach Anspruch 3, wobei die Vorrichtung zum Auslösen eines Alarms angepasst ist, wenn der Temperaturwert der Haut einen Maximalwert überschreitet.

5. Vorrichtung nach einem der vorstehenden Ansprüche, wobei das Elektrodensystem eine Vielzahl von Elektroden einer ersten Polarität (60, 62, 64, 66) umfasst, an eine der Polklemmen des Spannungsgenerators angeschlossen, und eine Vielzahl von Elektroden einer zweiten Polarität (68, 70, 72), an eine andere Polklemme des Spannungsgenerators angeschlossen, wobei die Elektroden einer ersten Polarität auf sich abwechselnde Weise mit den Elektroden der zweiten Polarität angeordnet sind und die Elektroden, die sich an den zwei Enden des Pflasters befinden, von gleicher Polarität sind.

6. Vorrichtung nach Anspruch 5, wobei der Abstand zwischen einer Anode und einer Kathode, die benachbart sind, kleiner ist als ein vorher festgelegter Abstand von zwei Zentimetern, damit die Stromleitungen nicht in die Hypodermis eindringen, sodass das Verursachen von Mikroläsionen der Muskeln oder Sehnen vermieden wird.

7. Vorrichtung nach einem der zwei vorstehenden Ansprüche, wobei jede der Elektroden, Anode oder Kathode, eine Vielzahl von Kontaktpunkten umfasst, sodass der auf mehrere Schnittstellen angelegte Strom verteilt und daher die lokale Stromdichte aufgeteilt wird.

8. Vorrichtung nach einem der Ansprüche 1 bis 7, verwendbar als lontophoresevorrichtung und auf einer Oberfläche des Pflasters (10), die dazu bestimmt ist, auf die Haut des Anwenders aufgetragen zu werden, eine Schicht von Wirkstoffen (14) beinhaltend, die mit dem Strom in die Haut des Anwenders einführbar sind.

9. Vorrichtung nach Anspruch 8, wobei die Schicht an Wirkstoffen in der Form von Hydrogel vorliegt.

## Claims

1. Electrostimulation and/or iontophoresis device comprising a patch (10) suitable for being applied onto the skin (12) of a user and a voltage generator (22) suitable for applying a low-intensity current through the skin of the user via a system of electrodes located in said patch;
said device comprising a self-regulator (16) that varies the voltage provided by said voltage generator according to the resistivity of the skin of the user under said patch, said device being **characterised in that** said self-regulator (16) comprises an element for measuring the resistance of the skin (24) and **in that** said self-regulator is suitable for using the value of the resistance in order to calculate, in real time, the value of the resistivity of the skin of the user under said patch.

2. Device according to the previous claim, said device being suitable for triggering an alarm when the value of the resistivity of the skin is not between a minimum value and a maximum value

3. Device according to any one of the previous claims, wherein said self-regulator (16) comprises an element for measuring the temperature of the skin (26).

4. Device according to claim 3, said device being suitable for triggering an alarm when the value of the temperature of the skin exceeds a maximum value.

5. Device according to any one of the previous claims, wherein said system of electrodes comprises a plurality of electrodes of a first polarity (60, 62, 64, 66) connected to one of the terminals of said voltage generator and a plurality of electrodes of the second polarity (68, 70, 72) connected to the other terminal of said voltage generator, said electrodes of a first polarity being positioned in an alternating manner with said electrodes of the second polarity and the electrodes located at the two ends of said patch being of the same polarity.

6. Device according to claim 5, wherein the distance between an anode and an adjacent cathode is less than a predetermined distance of two centimetres in order for the lines of current to not penetrate into the hypodermis in such a way as to avoid causing muscle or tendon microlesions.

7. Device according to any one of the previous two claims, wherein each of said electrodes, anode or cathode, comprises a plurality of points of contact in such a way as to distribute the applied current over a plurality of interfaces and thus divide the local current density.

8. Device according to any one of claims 1 to 7, usable as an iontophoresis device and comprising, on a surface of said patch intended to be applied onto the skin of the user, a layer of active substances (14) that can be introduced with the current into the skin of the user.

9. Device according to claim 8, wherein said layer of active substances is in the form of a hydrogel.
